# EUROPEAN PATENT SPECIFICATION

(11) **EP 4 540 730 B1**
(45) Date of publication and mention of the grant of the patent: **25.06.2025**
(21) Application number: 24732325.6
(22) Date of filing: 17.06.2024
(51) Int. Cl.: G16H 30/20, G16H 30/40, G16H 40/67, G16H 80/00, G06F 16/20, G06F 16/901, H04L 67/00, A61B 6/03, G06Q 50/00, H04L 67/12

(54) **AUTOMATIC CONFIGURATION OF MEDICAL SYSTEMS**
AUTOMATISCHE KONFIGURATION VON MEDIZINISCHEN SYSTEMEN
CONFIGURATION AUTOMATIQUE DE SYSTÈMES MÉDICAUX

(30) Priority: 22.06.2023 EP 23180923
(43) Date of publication of application: 23.04.2025
(73) Proprietor: Koninklijke Philips N.V., 5656 AG Eindhoven (NL)
(72) Inventor: JELFS, Sam Martin, 5656 AG Eindhoven (NL); VAN EE, Raymond, 5656 AG Eindhoven (NL); WESTERINK, Joanne Henriëtte Desirée Monique, 5656 AG Eindhoven (NL)
(74) Representative: Philips Intellectual Property & Standards
(86) International application number: PCT/EP2024/066712
(87) International publication number: WO 2024/260888

(56) References cited:
- EP-A1- 3 975 125
- WO-A1-2021/148145
- US-A1- 2016 110 434
- US-A1- 2016 179 864
- US-A1- 2021 042 589
- US-A1- 2022 382 803
- US-A1- 2023 177 057

## Description

### TECHNICAL FIELD

The invention relates to medical systems, in particular to the automated configuration of communication systems integrated into medical devices.

### BACKGROUND

Various types of medical systems such as medical imaging systems require great deals of training and experience to operate. Recently, telecommunication advances have begun to enable telemedicine and remote control of medical devices and medical imaging devices.
WO 2021/148145 A1 discloses an example of a method and a system configured to execute the method for intrusion detection to detect malicious insider threat activities within a network of multiple interconnected computerized user profiles, comprising the following method steps: training a Neural Network on multiple sets of user profile data for multiple user profiles and on multiple sets of activity data of the multiple user profiles of the network, such that the Neural Network is capable of predicting for future dates activities for multiple user profiles, and applying the trained Neural Network on the set of further user profile data of the further user profile, and predicting an activity of the further user profile based on the multiple sets of activity data by the trained Neural Network, and observing activity of the further user profile, and applying the trained Neural Network on the observed activity, and detecting malicious activity for the further user profile by the trained Neural Network, if the observed activity deviates from the predicted activity.
US 2021/042589 A1 discloses an example of a system and method for generating data visualizations using semantic graphs. The method includes generating an enriched data layer based on a plurality of knowledge graphs, the plurality of knowledge graphs including a plurality of first nodes, the enriched data layer including a plurality of second nodes, wherein each of the plurality of second nodes is connected via an edge to at least one of the plurality of first nodes; and generating a data visualization based on the enriched data layer and a request for data, wherein the request for data indicates a type of data corresponding to at least one of the plurality of second nodes, wherein the data visualization is generated using data represented by at least one of the plurality of first nodes connected to the at least one of the plurality of second nodes.

### SUMMARY

The invention provides for a medical system, a computer program, and a method as defined in the independent claims. Embodiments are given in the dependent claims.

In one aspect the invention provides for a medical system that comprises a memory storing machine-executable instructions and network graph. The network graph comprises network nodes and network edges that link the network nodes together. At least a portion of the network nodes comprise node-specific subject metadata. The network edges comprise configuration metadata. The configuration metadata comprises routing data configured for establishing a communication channel for communicating data between two network nodes of the network graph using a communication device. The medical system further comprises a computational system.

Execution of the machine-executable instructions causes the computational system to create a new node comprising new subject metadata in the network graph. Execution of the machine-executable instructions further causes the computational system to search for a closest matching network node selected from the network nodes. The closest matching network node is selected by comparing the new subject metadata to the node-specific subject metadata of the different nodes that make up the network graph. Execution of the machine-executable instructions further causes the computational system to construct new network edges for the new node in the network graph. The new network edges comprise a replication of at least a portion of the network edges of the closest matching network node.

In another aspect the invention provides for a method of operating a medical system. The medical system comprises a memory storing machine-executable instructions and a network graph. The network graph comprises network nodes and network edges that link the network nodes. At least a portion of the network nodes comprise node-specific subject metadata. The network edges comprise configuration metadata. The configuration metadata comprises routing data configured for establishing a communication channel for communicating data between two network nodes of the network graph using a communication device.

The method comprises creating a new node comprising new subject metadata in the network graph. The method further comprises searching for a closest matching network node selected from the network nodes. The closest matching network node is selected by comparing the new subject metadata to the node-specific metadata. The method further comprises constructing new network edges for the new node in the network graph. The new network edges comprise a replication of at least a portion of the network edges of the closest matching network node.

In another aspect, the invention provides for a computer program comprising machine-executable instructions for execution by a computational system. Execution of the machine-executable instructions causes the computational system to create a new node comprising new subject metadata in a network graph. The network graph comprises network nodes and network edges that link the network nodes. At least a portion of the network nodes comprise node-specific subject metadata. The network edges comprise configuration metadata. The configuration metadata comprises routing data configured for establishing a communication channel for communicating data between two network nodes of the network graph using a communication device.

Execution of the machine-executable instructions further causes the computational system to search for a closest matching network node selected from the network nodes. The closest matching network node is selected by comparing the new subject metadata to the new node-specific subject metadata. Execution of the machine-executable instructions further causes the computational system to construct new network edges for the new node in the network graph. The new network edges comprise a replication of at least a portion of the network edges of the closest matching network node.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 shows a flow chart which illustrates a method of using the medical system of Fig. 1.
Fig. 3 illustrates several examples of a network graph.
Fig. 4 illustrates a further example of a medical system.
Fig. 5 shows a flow chart which illustrates a method of using the medical system of Fig. 4.

### DESCRIPTION OF EMBODIMENTS

Like numbered elements in these Figures are either equivalent elements or perform the same function. Elements which have been discussed previously will not necessarily be discussed in later Figures if the function is equivalent.

Examples may be beneficial because it may provide for a means of automatically replicating routing data that is configured for establishing a communication channel. The routing data may conform in different examples. For example, the routing data may contain data such as a telephone number, an email address, an IP address, or other data which may be used to connect two communication devices together.

The closest matching network node may be determined using different criteria in different examples. In some examples the closest matching network nodes has the largest match with its node specific subject metadata and the new subject metadata of the new node. For example, the closest match could be determined by providing a score calculated by how many fields of the node specific subject metadata for a particular node matches the new subject metadata. A modification of this would be to have a weighted score which has weighting factors which emphasize particular fields having a closer match. The closest matching network node may also be generated as the average of a number of closely matching nodes using, for example, a k-nearest neighbors search approach.

In one example, the closest matching network node is identified by providing an identifier, classification, or specific metadata associated with the closest matching network. The closest matching network nodes is then found by searching for the identifier, classification, or specific metadata in the existing network nodes of the network graph. For example, a new member of staff (the new node) could just indicate a predecessor in the organization represented by the network graph. So, something like; "I am a new service engineer, please give me all the contacts that my colleague service engineers have as well", or "I succeed Mr. X, please give me all the contacts that Mr. X used to have". The current text has a high emphasis on automatically finding the closest match.

In some examples, the new subject metadata could be populated by a job role or position and the closest matching network node could be searched for as described in the paragraph above.

In some examples, the closest matching network node has edges that connect to other portions of the network graph. The new network edges are simply a replication or copying of these connections with the new node in place of the closest matching network node.

In another example, the medical system comprises a medical imaging system that is configured for acquiring medical imaging data. The medical system comprises the communication device. The subject-specific metadata comprises an image classification. The configuration metadata comprises device configuration data adapted for controlling the medical imaging system to acquire the medical imaging data. Execution of the machine-executable instructions further causes the computational system to select a chosen network edge from one of the network edges using the image classification. The new node represents the communication device of the medical imaging system. The chosen network edge is connected to an additional network edge representing a remote entity.

The image classification could for example be an identification of a particular type of anatomical structure or abnormal anatomical structure being present. The network edges could contain identifiers. When the identifier of a network edge matches the image classification, that network edge is then selected. This may for example be useful for automatically configuring the medical imaging system to image the anatomical structure specified by the identifier. In some examples, the medical system may display the image classification on a display for approval and/or correction before searching for the closest matching network node.

Execution of the machine-executable instructions further causes the computational system to establish the communication channel between the communication device and the remote entity using the routing data of the new network edge. The medical system is configured such that the communication channel enables at least partial control of the medical imaging system by the remote entity. Execution of the machine-executable instructions further causes the computational system to control the medical imaging system to acquire the medical imaging data using the device configuration data of the chosen network edge. This example may be beneficial because it may provide a means of both establishing a telecommunications link and controlling the medical system.

In another example execution of the machine-executable instructions further causes the computational system to send the image classification to the remote entity via the communication channel. Execution of the machine-executable instructions further causes the computational system to receive configuration modification commands from the remote entry via the communication channel. Execution of the machine-executable instructions further causes the computational system to modify the acquisition of the medical imaging data using the configuration modification commands. This example may be beneficial because it may provide for a means of automated or remote control of the medical system.

**In** another example, execution of the machine-executable instructions further causes the computational system to control the medical imaging system to acquire preliminary medical imaging data using preliminary configuration data. Execution of the machine-executable instructions further causes the computational system to receive the image classification in response to inputting the preliminary medical imaging data into an image classification neural network. Neural networks may be programmed to perform a classification of an image. For example, the image classification neural network may be a convolutional neural network or in some examples a U-Net neural network. The neural network may be trained by having training images that are labeled with the correct image classification.

In another example the medical imaging system is a magnetic resonance imaging system.

In another example the medical imaging system is an ultrasound imaging system.

In another example the medical imaging system is an X-ray system.

In another example the medical imaging system is a fluoroscope.

In another example the medical imaging system is a positron emission tomography system.

In another example the medical imaging system is a single-photon emission tomography system.

In another example the medical imaging system is a computed tomography system.

In another example the medical imaging system is a telemedicine system.

In another example the medical imaging system is a hospital information system.

In another example the medical imaging system is a medical diagnostic machine. A medical diagnostic machine as used herein is a laboratory instrument used to perform medical tests or assays.

In another example the configuration metadata comprises work processes and workflows.

In another example the configuration metadata comprises a flag indicating if the configuration metadata is private or non-private. The execution of the machine-executable instructions further causes the computational system to exclude network edges of the closest matching network node from replicated if the flag is set to private. This example may be beneficial because it may provide for higher security because the leak of confidential or private data is secured and is not allowed to be copied when a new node is added.

In another example the subject-specific metadata comprises a job role.

In another example the subject-specific metadata comprises functional requirements fulfilled by the subject.

In another example, execution of the machine-executable instructions further causes the computational system to receive a selection of a cancelled network node. The cancelled network node is one of the network nodes. Execution of the machine-executable instructions further causes the computational system to send a pre-recorded message to network nodes connected in the cancelled network node via the communication channel for network nodes connected to the cancelled network node via the network edges. Execution of the machine-executable instructions further causes the computational system to remove the cancelled network nodes and network edges connected to the cancelled network node from the network graph. This example may be beneficial because it may provide for an automated means of editing and removing nodes that represent a particular subject. Using the routing data other users or objects in the database may be warned that a particular user is no longer part of the network graph.

In another example the configuration metadata comprises usage frequency data. The network edges of the matching network node with usage data below a predetermined usage threshold are excluded from being replaced. This may be beneficial because this may represent sparsely used data will not be wasted when it is transferred and then stored in another location.

In another example, execution of the machine-executable instructions further causes the computational system to receive multiple network nodes comprising created subject metadata. Execution of the machine-executable instructions further causes the computational system to iteratively add the multiple network nodes to a new network graph. The iteratively adding of the multiple network nodes comprises adding one of the multiple network nodes to the new network graph and copying the network edges of the network graph to the new network graph by matching the created subject metadata to the subject metadata of the network graph. This example may be beneficial because it may provide a means of taking the structure of a previous network graph and then using it to construct a new network graph. This may for example be useful in situations where the network graph for a hospital is for example used to create a new network graph in a new hospital or new location.

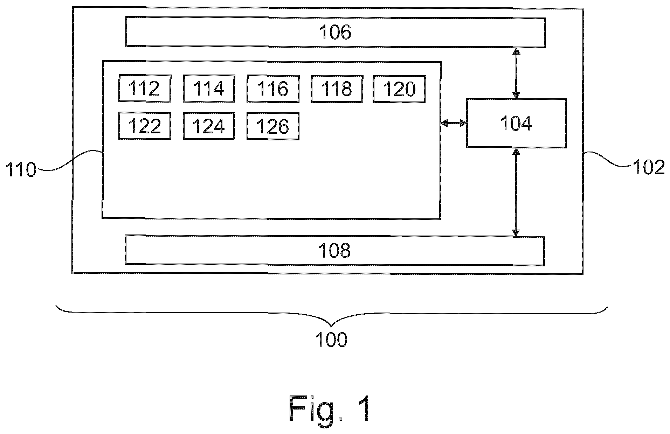

Fig. 1 illustrates an example of a medical system 100. The medical system 100 is shown as comprising a computer 102 that has a computational system 104. The computational system 104 is in communication with an optional hardware interface 106. The computational system 104 is in further communication with a communication device 108. The communication device 108 could for example be a network interface.

The computer 102 is further shown as comprising a memory 110 that is also in communication with the computational system 104. The memory 110 is intended to represent various types of memory which are accessible to the computational system 104.

The memory 110 is shown as comprising machine-executable instructions 112 which enable the computational system 104 to control the other components of the medical system 100 as well as perform various data processing and numerical tasks. The memory 110 is shown as further containing a network graph 114. The network graph comprises network nodes and network edges that link the network nodes together. At least a portion of the network nodes comprise the node-specific subject metadata. The network edges comprise configuration metadata. The configuration metadata comprises routing data configured for establishing a communication channel for communicating data between two network nodes of the network graph using the communication device 108.

The memory 110 is further shown as containing a new node 116 to be added to the network graph 114. The new node 116 comprises new subject metadata 118. The memory 110 is shown as comprising a comparison module 120 that is able to compare the new subject metadata 118 with node-specific subject metadata of the rest of the network graph 114. This may for example find the node with the closest values for its metadata or it may be a weighted average of which one is closer.

The memory 110 is further shown as containing a closest-matching network node 122 that was selected from the network nodes of the network graph 114. The memory 110 is then shown as also containing the network edges of the closest-matching network node 124. The memory 110 is then shown as containing new network edges 126 that were replications of the network edges of the closest-matching network node 124. In some cases, some of the network edges 124 may be left out. For example, there may be a flag to indicate that a network connection is personal or there may be other data used to exclude certain of the network edges 124. The new node 116 is then added to the network graph 114 by connecting the new node 116 using the new network edges 126. The new network edges 126 originate or are connected to the new node 116 and whatever nodes the closest-matching network node 122 was connected to.

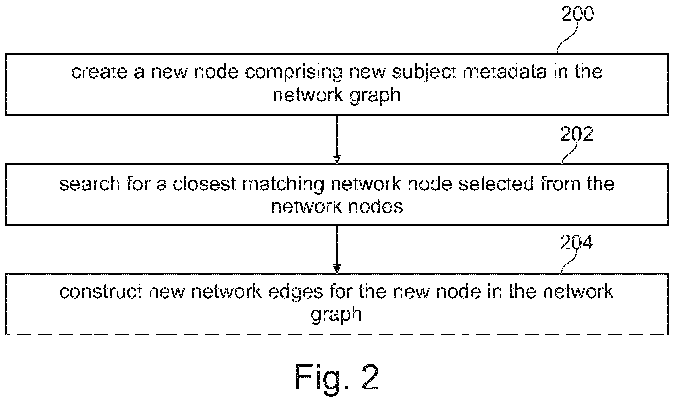

Fig. 2 shows a flowchart which illustrates a method of operating the medical system 100 of Fig. 1. In step 200 a new node 116 is created. The new node comprises new subject metadata 118. In step 202 the closest-matching network node 122 is searched for within the network nodes of the network graph 114. In step 204 the new network edges 126 for the new node 116 are constructed in the network graph by replicating at least some of the network edges 124 of the closest-matching network node 122.

When new users are added to a communication system, they may be presented with either an empty contact list or a contact list prefilled with persons within their company. Often times this is not useful to the new user and gives no context to their contacts. As an example, if the user needs to find technical support for a specific device or procedure searching their contact list may produce many results and it is difficult to know who they should speak with. This often drives the user to need to search on other inter-/intra-net sites to find additional contextual information first or ask for referrals from other employees to identify the best contact to assist them. Examples may apply network theory at a company-wide or even between-company level to determine what and why certain persons have each other in their contact lists, and to then automatically generate a contact list when a new person is entered into the network.

Network theory is a version of graph theory where both the nodes and the edges of the graph have data associated with them. It has been used for some time in the analysis of social networks, and to understand the relationships between social entities within a network. In the case of a contacts list system each person can be considered a node in the graph, and the edge between nodes is the existence of one or both persons having the other in their contact list.

When a new person joins an organization, they are often confronted by an empty email address book, with little to no information on who the best contacts are for their role within a company. The invention aims to automatically, and adaptively, construct a network of connections between people.

Some examples may provide one or more of the following features:
- A system capable of representing persons as nodes within a network graph.
   ∘ Each node has a set of metadata associated with it that represents the person, in terms of job role and specific functional requirements
   ∘ Edges that link nodes representing the existence of an entry in one or both nodes associated contact lists.
      ∘ Metadata associated with the edge that represents the reason for the edge existence.
- A method to analyze the graph and to automatically create / suggest new edges between existing nodes.
- A method to automatically create edges when a new node is added to the network with no pre-existing edges.
- A method to create a new network with a completely new set of nodes, and use the existing networks metadata to create edges.

As a concrete example, a network graph may be constructed where each node of the graph represents an individual person (or functional person) with associated metadata, for example:

```
Class::node{
                uid: <unique reference number>
                name: <Name of the person>
                job_family: <broad job category>
                job_role: <more specific job role>
                keywords: <comma-separated list of keywords for person>
                
```

Each edge of the graph represents a link between two people, for example:

```
Class::edge{
                uid: <unique reference number>
                nodeA: <uid of first node>
                nodeB: <uid of second node>
                connectionDirection: <direction of edge, AB, BA, ABA>
 ...
 }
```

When a new person (node) is entered into the graph the metadata is created that describes that person. By searching the graph for existing people with similar metadata as the new person their existing connections can be used to create or suggest connections for the new person.

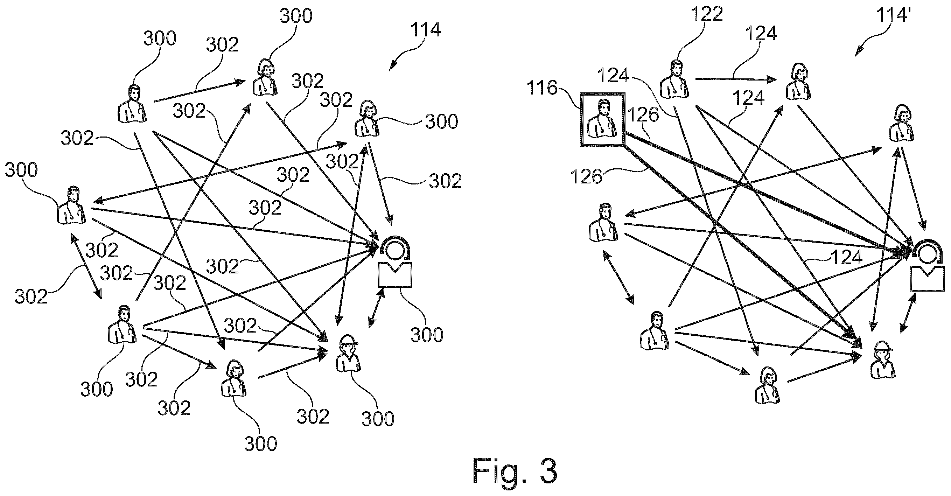

Fig. 3 shows two different views of the network graph 114. The network graph 114 illustrates an example of the original network graph 114. The network graph 114' is the network graph 114 after the new node 116 has been added. The network graph 114 is shown as comprising network nodes 300 and a system of network edges 302 that are used to connect the network nodes 300. Some of the network edges 302 indicate a bidirectional connection and some of the network nodes indicate a directed connection or a one-way connection.

In the modified network graph 114 the new node 116 and the closest-matching network node 122 are identified. The closest-matching network node 122 is connected to the network edges 124 of the closest-matching network node 122. The closest-matching network node 122 has four network edges 124. The new network edges 126 were constructed by taking just two of those and then connecting the new node 116 using these same connections 126. Various metadata or flags could be set in the metadata of the network edges 124 to enable their selection or deselection.

In another example the system is extended such that the metadata associated with all nodes and edges is anonymized sufficiently that a new network can be created with only node information available, and the edge information from the first network will be used to construct the edges of the second network.

In another example additional metadata can be added to the edge connections that specifies if the edge was manually or automatically created, with manually created edges given a different importance function, which will affect the likelihood of their suggestion when adding a new node.

In a further example the system is extended to be able to track the frequency of use of each edge connection, via email, voice calling, text messaging service, etc. such that the edge metadata can be extended with a weighting function for the frequency of use.

In a further example the system such that the edge connection and node metadata also include information related to the business processes or workflows. For instance, that flow could be from role A to role B to role C, and the system could then be configured to limit the new prefilled contacts to maximum numbers based on the new function, such that for instance a new person in role A gets a maximum of 50 B persons prefilled, but only the top 10 C roles. Whereas a new person in role B would get much more people in role C prefilled.

In a further example the system is extended such that users are able to manually specify additional metadata for edge connections, such as if the connection is private, and should not be automatically suggested for others; or of high relevance even if infrequently used such that it should have a higher importance weighting.

In a further example meatadata on previous job roles, inside and outside of the network may be added to suggest contacts that may be useful not due to their current job role, but for previous knowledge and experience.

In a further example the system can be extended to work across networks where a network may represent a single organization to allow for connections between organizations, this can be especially useful for job roles such as technical support persons.

In a further example the system may be extended such that if a person leaves an organization and is replaced by a new person, then all connected nodes from the first person can be automatically notified of the change of person and the update of the connection information. This can also apply when the person changes job role within the organization.

In a further example based on the second embodiment, analysis of many networks representing different organizations may allow for clustering the organizations by type. When a new network is created the organization can be characterized and the metadata for only other organizations of a similar type will be used for the generation of the new network.

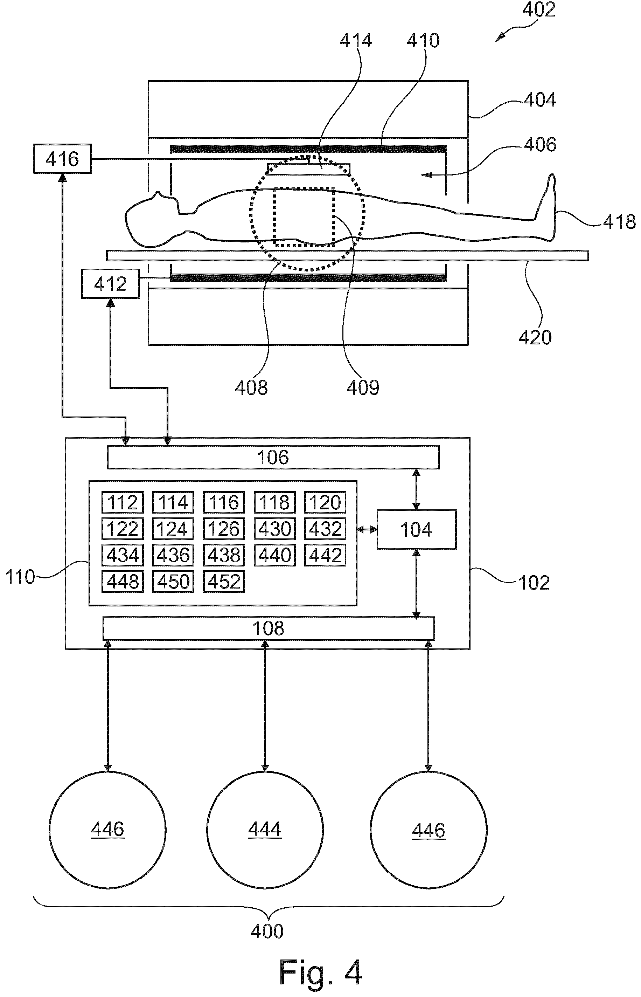

Fig. 4 illustrates a further example of a medical system 400. It is intended to represent a telemedicine system or a remote-controlled medical imaging system. In this example the example is explained in the context of a magnetic resonance imaging system 402. However, other types of medical imaging systems such as computed tomography or ultrasound or other types of medical imaging systems could also be substituted.

The magnetic resonance imaging system 402 comprises a magnet 404. The magnet 404 is a superconducting cylindrical type magnet with a bore 406 through it. It is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject. The arrangement of the two sections area is similar to that of a Helmholtz coil. Open magnets are popular because the subject is less confined. Inside the cryostat of the cylindrical magnet there is a collection of superconducting coils.

Within the bore 406 of the cylindrical magnet 404 there is an imaging zone 408 where the magnetic field is strong and uniform enough to perform magnetic resonance imaging. A field of view 409 is shown within the imaging zone 408. The k-space data are acquired for the field of view 409. The region of interest could be identical with the field of view 409 or it could be a sub volume of the field of view 409. A subject 418 is shown as being supported by a subject support 420 such that at least a portion of the subject 418 is within the imaging zone 408 and the field of view 409.

Within the bore 406 of the magnet there is also a set of magnetic field gradient coils 410 which is used for the acquisition of measured k-space data to spatially encode magnetic spins within the imaging zone 408 of the magnet 404. The magnetic field gradient coils 410 are connected to a magnetic field gradient coil power supply 412. The magnetic field gradient coils 410 are intended to be representative. Typically, magnetic field gradient coils 410 contain three separate sets of coils for spatial encoding in three orthogonal spatial directions. A magnetic field gradient power supply supplies current to the magnetic field gradient coils. The current supplied to the magnetic field gradient coils 410 is controlled as a function of time and may be ramped or pulsed.

Adjacent to the imaging zone 408 is a radio frequency coil 414 for manipulating the orientations of magnetic spins within the imaging zone 408 and for receiving radio transmissions from spins also within the imaging zone 408. The radio frequency antenna may contain multiple coil elements. The radio frequency antenna may also be referred to as a channel or antenna. The radio frequency coil 414 is connected to a radio frequency transceiver 416. The radio frequency coil 414 and radio frequency transceiver 416 may be replaced by separate transmit and receive coils and a separate transmitter and receiver. It is understood that the radio frequency coil 414 and the radio frequency transceiver 416 are representative. The radio frequency coil 414 is intended to also represent a dedicated transmit antenna and a dedicated receive antenna. Likewise, the transceiver 416 may also represent a separate transmitter and receiver. The radio frequency coil 414 may also have multiple receive/transmit elements and the radio frequency transceiver 416 may have multiple receive/transmit channels. The transceiver 416 and the gradient controller 412 are shown as being connected to the hardware interface 106 of the computer system 102.

In this example, the node 116 that is added represents a clinical procedure or new subject being added to a medical database. The matching the new subject metadata 118 with the closest-matching network node 122 is used to select controls for the magnetic resonance imaging system automatically. This metadata could include such things as the type of procedure desired as well as the metadata descriptive of the subject 418. For example, some magnetic resonance imaging protocols are affected by the size and weight of the subject 418.

In some examples, preliminary pulse sequence commands 430 are contained within the memory 110. The computational system 104 uses these to acquire preliminary k-space data 432. This for example could be scout or survey k-space data. The memory 110 is then further shown as containing a scout or survey image 434 that has been reconstructed from the preliminary k-space data 432. The memory 110 is optionally shown as containing an image classifier neural network 436. This could for example be a convolutional neural network that is used to classify or trained to classify scout images 434. The memory 110 is further shown as containing an image classification 438 that has been received in response to inputting the scout image 434 into the image classifier neural network 436. This may for example be used to identify various types of pathologies or locations that have been imaged within the subject 418. In any case, this image classification 438 is used to select one of the new network edges 126.

When the new network edge 126 is selected, this is equivalent to selecting a remote entity 444 to connect to and selected pulse sequence commands 442. This then selects the specific pulse sequence commands to use to acquire additional data as well as automatically connecting to the remote entity 444. The medical system 400 is shown as comprising a remote entity 444 and several other remote entities 446. The remote entity 444 could be a connection with a physician such as is used for telemedicine, or it may also be an automatic control system such as a robotic computer program which is used to control the magnetic resonance imaging system 402 remotely and automatically. The memory 110 is further shown as containing configuration modification commands 448 received from the remote entity 444. These are used to modify the selected pulse sequence commands 442. After modification the selected pulse sequence commands 442 are used to acquire clinical k-space data 450. The selected pulse sequence commands 442 are used by the computational system 104 to control the operation of the magnetic resonance imaging system to acquire the clinical k-space data. The clinical k-space data 450 is then used to reconstruct the clinical magnetic resonance image 452.

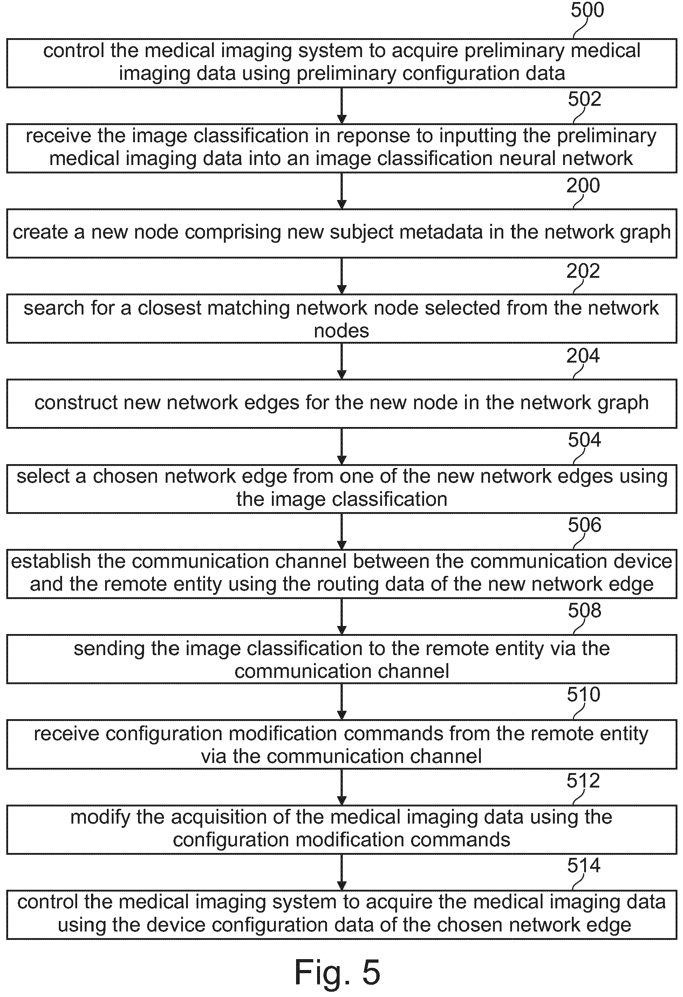

Fig. 5 shows a flowchart which illustrates a method of using the medical system 400 of Fig. 4. Some of these steps in this flowchart can be performed in a different order. For example, steps 500 and 502 are performed before steps 200, 202, and 204. In other examples steps 200, 202, and 204 are performed before steps 500 and 502.

In step 500 the medical imaging system or magnetic resonance imaging system 402 is controlled to acquire the preliminary medical imaging data, which in this case is the preliminary k-space data 432, using the preliminary pulse sequence commands 430. The preliminary pulse sequence commands 430 are the preliminary configuration data. In step 502 the image classification 432 is received in response to inputting the scout image 434 into the image classification neural network. Next, steps 200, 202, and 204 are performed as was illustrated in Fig. 2. In this example the new node represents the subject 418 and any metadata descriptive of the subject or a selection of a particular type of magnetic resonance imaging protocol. The selection of the closest-matching neural network node 122 and its network edges 124 represent magnetic resonance imaging protocols that are available to image the subject 418.

In step 504 the image classification 438 is used to select a chosen network edge from the new network edges 126. This provides the selected pulse sequence commands 442 and establishes a communication link or data for establishing the communication link with the remote entity 444. In step 508, the image classification 438 is sent to the remote entity 444. In step 510 the configuration modification commands 448 are received from the remote entity 444. In step 512 the acquisition of the medical imaging data is modified using the configuration modification commands. In this particular example this is done by modifying the selected pulse sequence commands 442 with the configuration modification commands 448. Then, in step 514, the medical imaging system, or in this case the magnetic resonance imaging system 402, is controlled using the device configuration of the chosen network edge, which in this example is the modified selected pulse sequence commands 442.

It is understood that one or more of the aforementioned examples may be combined as long as the combined examples are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. **In** some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. **In** other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. **In** the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, **MIDI** interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Medical imaging data is defined herein as being recorded measurements made by a medical imaging system, such as a tomographic medical imaging system, that descriptive of an internal anatomy of a subject. The medical imaging data may be reconstructed into a medical image. A medical image is defined herein as being the reconstructed two- or three-dimensional visualization of anatomic data contained within the medical imaging data. This visualization can be performed using a computer.

K-space data is defined herein as being the recorded measurements of radio frequency signals emitted by atomic spins using the antenna of a Magnetic resonance apparatus during a magnetic resonance imaging scan. Magnetic resonance data is an example of medical image data.

A Magnetic Resonance Imaging (MRI) image or MR image is defined herein as being the reconstructed two- or three-dimensional visualization of anatomic data contained within the magnetic resonance imaging data. This visualization can be performed using a computer.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

100 medical system
102 computer
104 computational system
106 hardware interface
108 communication device (network interface)
110 memory
112 machine executable instructions
114 network graph
114' modified network graph
116 new node
118 new subject metadata
120 comparison module
122 closest matching network node
124 network edges of closest matching network node
126 new network edges
200 create a new node comprising new subject metadata in the network graph
202 search for a closest matching network node selected from the network nodes
204 construct new network edges for the new node in the network graph
300 network nodes
302 network edges
400 medical system
402 magnetic resonance imaging system (medical imaging system)
404 magnet
406 bore of magnet
408 imaging zone
409 field of view
410 magnetic field gradient coils
412 magnetic field gradient coil power supply
414 radio frequency coil
416 transceiver
418 subject
420 subject support
430 preliminary pulse sequence commands
432 preliminary k-space data (preliminary medical imaging data)
434 scout image
436 image classification neural network
438 image classification
442 selected pulse sequence commands
444 remote entity
446 other remote entities
448 configuration modification commands
450 clinical k-space data (medical imaging data)
452 clinical magnetic resonance image
500 control the medical imaging system to acquire preliminary medical imaging data using preliminary configuration data
502 receive the image classification in response to inputting the preliminary medical imaging data into an image classification neural network
504 select a chosen network edge from one of the new network edges using the image classification
506 establish the communication channel between the communication device and the remote entity using the routing data of the new network edge
508 sending the image classification to the remote entity via the communication channel
510 receive configuration modification commands from the remote entity via the communication channel
512 modify the acquisition of the medical imaging data using the configuration modification commands
514 control the medical imaging system to acquire the medical imaging data using the device configuration data of the chosen network edge

## Claims

1. A medical system (100, 400) comprising:
- a memory (110) storing machine executable instructions (112) and a network graph (114, 114'), wherein the network graph comprises network nodes (300) and network edges (302) that link the network nodes, wherein at least a portion of the network nodes comprise node specific subject metadata, wherein the network edges comprise configuration metadata, wherein the configuration metadata comprises routing data configured for establishing a communication channel for communicating data between two network nodes of the network graph using a communication device;
- a computational system (104), wherein execution of the machine executable instructions causes the computational system to:
- create (200) a new node (116) comprising new subject metadata (118) in the network graph;
- search (202) for a closest matching network node (122) selected from the network nodes, wherein the closest matching network node is selected by comparing the new subject metadata to the node specific subject metadata,
- construct (204) new network edges (126) for the new node in the network graph, wherein the new network edges comprise a replication of at least a portion of the network edges of the closest matching network node.

2. The medical system of claim 1, wherein the medical system comprises a medical imaging system (402) configured for acquiring medical imaging data (450), wherein the medical system comprises the communication device, wherein the subject specific metadata comprises an image classification (438), wherein the configuration metadata comprises device configuration data adapted for controlling the medical imaging system to acquire the medical imaging data, wherein execution of the machine executable instructions further causes the computational system to:
- select (504) a chosen network edge from one of the new network edges using the image classification, wherein the new node represents the communication device of the medical imaging system, wherein the chosen network edge is connected to an additional network edge representing a remote entity (444);
- establish (506) the communication channel between the communication device and the remote entity using the routing data of the new network edge, wherein the medical system is configured such that the communication channel enables at least partial control of the medical imaging system by the remote entity;
- control (514) the medical imaging system to acquire the medical imaging data using the device configuration data of the chosen network edge.

3. The medical system of claim 2, wherein execution of the machine executable instructions further causes the computational system to:
- sending (508) the image classification to the remote entity via the communication channel;
- receive (510) configuration modification commands (448) from the remote entity via the communication channel; and
- modify (512) the acquisition of the medical imaging data using the configuration modification commands.

4. The medical system of claim 2 or 3, wherein execution of the machine executable instructions further causes the computational system to:
- control (500) the medical imaging system to acquire preliminary medical imaging data (432) using preliminary configuration data;
- receive (502) the image classification in response to inputting the preliminary medical imaging data into an image classification neural network (436).

5. The medical system of any one of claims 2, 3, or 4, wherein the medical imaging system is any one of the following: a magnetic resonance imaging system (402), ultrasound imaging system, an X-ray system, a fluoroscope, a positron emission tomography system, a single photon emission tomography system, and a computed tomography system.

6. The medical system of any one of the preceding claims, wherein the medical system is a telemedicine system.

7. The medical system of any one of claims 1 through 5, wherein the medical system is a hospital information system or a medical diagnostic machine.

8. The medical system of any one of the preceding claims, wherein the configuration metadata comprises any one of the following: work processes and workflows.

9. The medical system of any one of the preceding claims, wherein the configuration metadata comprises a flag indicating if the configuration metadata is private or non-private, wherein execution of the machine executable instructions further cause the computational system to exclude network edges of the closest matching network node from being replicated if they have a private indication.

10. The medical system of any one of the preceding claims, wherein the subject specific metadata comprises a job role and/or functional requirements.

11. The medical system of any one of the preceding claims, wherein execution of the machine executable instructions further causes the computational system to:
- receive a selection of a cancelled network node, wherein the cancelled network node is one of the network nodes;
- send a prerecorded message to network nodes connected to the cancelled network node via the communication channel for network nodes connected to the cancelled network node via network edges; and
- remove the cancelled network node and network edges connected to the cancelled network node from the network graph.

12. The medical system of any one of the preceding claims, wherein the configuration metadata comprises usage frequency data, wherein the network edges of the matching network node with usage frequency data below a predetermined usage threshold are excluded from being replicated.

13. The medical system of any one of the preceding claims, wherein execution of the machine executable instructions further causes the computational system to:
- receive multiple network nodes comprising created subject metadata;
- iteratively add the multiple network nodes to a new network graph, wherein iteratively adding the multiple network nodes comprises:
- adding one of the multiple network nodes to the new network graph,
- copying network edges of the network graph to the new network graph by matching the created subject metadata to the subject metadata of the network graph.

14. A method of operating a medical system (100, 400), wherein the medical system comprises a memory (110) storing machine executable instructions (112) and a network graph (114, 114'), wherein the network graph comprises network nodes (300) and network edges (302) that link the network nodes, wherein at least a portion of the network nodes comprise node specific subject metadata, wherein the network edges comprise configuration metadata, wherein the configuration metadata comprises routing data configured for establishing a communication channel for communicating data between two network nodes of the network graph using a communication device, wherein the method comprises:
- creating (200) a new node (116) comprising new subject metadata (118) in the network graph;
- searching (202) for a closest matching network node (122) selected from the network nodes, wherein the closest matching network node is selected by comparing the new subject metadata to the node specific subject metadata,
- constructing (204) new network edges (126) for the new node in the network graph, wherein the new network edges comprise a replication of at least a portion of the network edges of the closest matching network node.

15. A computer program product comprising machine executable instructions (112) for execution by a computational system (104), wherein execution of the machine executable instructions causes the computational system to:
- create (200) a new node (116) comprising new subject metadata (118) in a network graph, wherein the network graph comprises network nodes (300) and network edges (302) that link the network nodes, wherein at least a portion of the network nodes comprise node specific subject metadata, wherein the network edges comprise configuration metadata, wherein the configuration metadata comprises routing data configured for establishing a communication channel for communicating data between two network nodes of the network graph using a communication device;
- search (202) for a closest matching network node (122) selected from the network nodes, wherein the closest matching network nodes is selected by comparing the new subject metadata to the node specific subject metadata,
- construct (204) new network edges (126) for the new node in the network graph, wherein the new network edges comprise a replication of at least a portion of the network edges of the closest matching network node.

## Patentansprüche

1. Medizinisches System (100, 400), umfassend:
- einen Speicher (110), der maschinenausführbare Anweisungen (112) und einen Netzwerkgraphen (114, 114') speichert, wobei der Netzwerkgraph Netzwerkknoten (300) und Netzwerkkanten (302) umfasst, die die Netzwerkknoten verknüpfen, wobei mindestens ein Teil der Netzwerkknoten knotenspezifische Subjektmetadaten umfasst, wobei die Netzwerkkanten Konfigurationsmetadaten umfassen, wobei die Konfigurationsmetadaten Routingdaten umfassen, die zum Einrichten eines Kommunikationskanals zum Übermitteln von Daten zwischen zwei Netzwerkknoten des Netzwerkgraphen unter Verwendung einer Kommunikationsvorrichtung konfiguriert sind;
- ein Rechensystem (104), wobei Ausführung der maschinenausführbaren Anweisungen das Rechensystem zu Folgendem veranlasst:
- Erstellen (200) eines neuen Knotens (116), der neue Subjektmetadaten (118) umfasst, in dem Netzwerkgraphen;
- Suchen (202) nach einem aus den Netzwerkknoten ausgewählten nächstgelegenen übereinstimmenden Netzwerkknoten (122), wobei der nächstgelegene übereinstimmende Netzwerkknoten durch Vergleichen der neuen Subjektmetadaten mit den knotenspezifischen Subjekmetadaten ausgewählt wird,
- Konstruieren (204) neuer Netzwerkkanten (126) für den neuen Knoten in dem Netzwerkgraphen, wobei die neuen Netzwerkkanten eine Replikation von mindestens einem Teil der Netzwerkkanten des nächstgelegenen übereinstimmenden Netzwerkknotens umfassen.

2. Medizinisches System nach Anspruch 1, wobei das medizinische System ein medizinisches Bildgebungssystem (402) umfasst, das zum Erfassen medizinischer Bildgebungsdaten (450) konfiguriert ist, wobei das medizinische System die Kommunikationsvorrichtung umfasst, wobei die subjektspezifischen Metadaten eine Bildklassifizierung (438) umfassen, wobei die Konfigurationsmetadaten Vorrichtungskonfigurationsdaten umfassen, die zum Steuern des medizinischen Bildgebungssystems zum Erfassen der medizinischen Bildgebungsdaten angepasst sind, wobei Ausführung der maschinenausführbaren Anweisungen das Rechensystem weiter zu Folgendem veranlasst:
- Auswählen (504) einer gewählten Netzwerkkante aus einer der neuen Netzwerkkanten unter Verwendung der Bildklassifizierung, wobei der neue Knoten die Kommunikationsvorrichtung des medizinischen Bildgebungssystems darstellt, wobei die gewählte Netzwerkkante mit einer zusätzlichen Netzwerkkante verbunden ist, die eine entfernte Entität (444) darstellt;
- Einrichten (506) des Kommunikationskanals zwischen der Kommunikationsvorrichtung und der entfernten Entität unter Verwendung der Routingdaten der neuen Netzwerkkante, wobei das medizinische System derart konfiguriert ist, dass der Kommunikationskanal mindestens eine teilweise Steuerung des medizinischen Bildgebungssystems durch die entfernte Entität ermöglicht;
- Steuern (514) des medizinischen Bildgebungssystems zum Erfassen der medizinischen Bildgebungsdaten unter Verwendung der Vorrichtungskonfigurationsdaten der gewählten Netzwerkkante.

3. Medizinisches System nach Anspruch 2, wobei Ausführung der maschinenausführbaren Anweisungen das Rechensystem weiter zu Folgendem veranlasst:
- Senden (508) der Bildklassifizierung über den Kommunikationskanal an die entfernte Entität;
- Empfangen (510) von Konfigurationsmodifikationsbefehlen (448) von der entfernten Entität über den Kommunikationskanal; und
- Modifizieren (512) der Erfassung der medizinischen Bildgebungsdaten unter Verwendung der Konfigurationsmodifikationsbefehle.

4. Medizinisches System nach Anspruch 2 oder 3, wobei Ausführung der maschinenausführbaren Anweisungen das Rechensystem weiter zu Folgendem veranlasst:
- Steuern (500) des medizinischen Bildgebungssystems, um vorläufige medizinische Bildgebungsdaten (432) unter Verwendung vorläufiger Konfigurationsdaten zu erfassen;
- Empfangen (502) der Bildklassifizierung als Reaktion auf Eingeben der vorläufigen medizinischen Bildgebungsdaten in ein neuronales Bildklassifizierungsnetzwerk (436).

5. Medizinisches System nach einem der Ansprüche 2, 3 oder 4, wobei das medizinische Bildgebungssystem ein beliebiges der Folgenden ist: ein Magnetresonanzbildgebungssystem (402), ein Ultraschallbildgebungssystem, ein Röntgensystem, ein Fluoroskop, ein Positronen-Emissions-Tomographiesystem, ein Einzelphotonen-Emissions-Tomographiesystem und ein Computertomographiesystem.

6. Medizinisches System nach einem der vorstehenden Ansprüche, wobei das medizinische System ein Telemedizinsystem ist.

7. Medizinisches System nach einem der Ansprüche 1 bis 5, wobei das medizinische System ein Krankenhausinformationssystem oder ein medizinisches Diagnosegerät ist.

8. Medizinisches System nach einem der vorstehenden Ansprüche, wobei die Konfigurationsmetadaten einen beliebigen des Folgenden umfassen: Arbeitsprozesse und Arbeitsabläufe.

9. Medizinisches System nach einem der vorstehenden Ansprüche, wobei die Konfigurationsmetadaten ein Flag umfassen, das angibt, ob die Konfigurationsmetadaten privat oder nicht privat sind, wobei Ausführung der maschinenausführbaren Anweisungen das Rechensystem weiter veranlasst, Netzwerkkanten des nächstgelegenen übereinstimmenden Netzwerkknotens von der Replikation auszuschließen, wenn diese eine Privatangabe aufweisen.

10. Medizinisches System nach einem der vorstehenden Ansprüche, wobei die subjektspezifischen Metadaten eine Berufsrolle und/oder funktionale Anforderungen umfassen.

11. Medizinisches System nach einem der vorstehenden Ansprüche, wobei Ausführung der maschinenausführbaren Anweisungen das Rechensystem weiter zu Folgendem veranlasst:
- Empfangen einer Auswahl eines deaktivierten Netzwerkknotens, wobei der deaktivierte Netzwerkknoten einer der Netzwerkknoten ist;
- Senden einer vorab aufgezeichneten Nachricht an Netzwerkknoten, die mit dem deaktivierten Netzwerkknoten verbunden sind, über den Kommunikationskanal für Netzwerkknoten, die über Netzwerkkanten mit dem deaktivierten Netzwerkknoten verbunden sind; und
- Entfernen des deaktivierten Netzwerkknotens und der mit dem deaktivierten Netzwerkknoten verbundenen Netzwerkkanten aus dem Netzwerkgraphen.

12. Medizinisches System nach einem der vorstehenden Ansprüche, wobei die Konfigurationsmetadaten Nutzungshäufigkeitsdaten umfassen, wobei die Netzwerkkanten des übereinstimmenden Netzwerkknotens mit Nutzungshäufigkeitsdaten unterhalb eines vorbestimmten Nutzungsschwellenwerts von der Replikation ausgeschlossen sind.

13. Medizinisches System nach einem der vorstehenden Ansprüche, wobei Ausführung der maschinenausführbaren Anweisungen das Rechensystem weiter zu Folgendem veranlasst:
- Empfangen multipler Netzwerkknoten, die erstellte Subjektmetadaten umfassen;
- iteratives Hinzufügen der multiplen Netzwerkknoten zu einem neuen Netzwerkgraphen, wobei iteratives Hinzufügen der multiplen Netzwerkknoten Folgendes umfasst:
- Hinzufügen eines der multiplen Netzwerkknoten zu dem neuen Netzwerkgraphen,
- Kopieren von Netzwerkkanten des Netzwerkgraphen in den neuen Netzwerkgraphen durch Abgleichen der erstellten Subjektmetadaten mit den Subjektmetadaten des Netzwerkgraphen.

14. Verfahren zum Betreiben eines medizinischen Systems (100, 400), wobei das medizinische System einen Speicher (110) umfasst, der maschinenausführbare Anweisungen (112) und einen Netzwerkgraphen (114, 114') speichert, wobei der Netzwerkgraph Netzwerkknoten (300) und Netzwerkkanten (302) umfasst, die die Netzwerkknoten verknüpfen, wobei mindestens ein Teil der Netzwerkknoten knotenspezifische Subjektmetadaten umfasst, wobei die Netzwerkkanten Konfigurationsmetadaten umfassen, wobei die Konfigurationsmetadaten Routingdaten umfassen, die zum Einrichten eines Kommunikationskanals zum Übermitteln von Daten zwischen zwei Netzwerkknoten des Netzwerkgraphen unter Verwendung einer Kommunikationsvorrichtung konfiguriert sind, wobei das Verfahren umfasst:
- Erstellen (200) eines neuen Knotens (116), der neue Subjektmetadaten (118) umfasst, in dem Netzwerkgraphen;
- Suchen (202) nach einem nächstgelegenen übereinstimmenden Netzwerkknoten (122), der aus den Netzwerkknoten ausgewählt wird, wobei der nächstgelegene übereinstimmende Netzwerkknoten durch Vergleichen der neuen Subjektmetadaten mit den knotenspezifischen Subjektmetadaten ausgewählt wird,
- Konstruieren (204) neuer Netzwerkkanten (126) für den neuen Knoten in dem Netzwerkgraphen, wobei die neuen Netzwerkkanten eine Replikation von mindestens einem Teil der Netzwerkkanten des nächstgelegenen übereinstimmenden Netzwerkknotens umfassen.

15. Computerprogramm, das maschinenausführbare Anweisungen (112) zur Ausführung durch ein Rechensystem (104) umfasst, wobei Ausführung der maschinenausführbaren Anweisungen das Rechensystem zu Folgendem veranlasst:
- Erstellen (200) eines neuen Knotens (116), der neue Subjektmetadaten (118) umfasst, in einem Netzwerkgraphen, wobei der Netzwerkgraph Netzwerkknoten (300) und Netzwerkkanten (302) umfasst, die die Netzwerkknoten verknüpfen, wobei mindestens ein Teil der Netzwerkknoten knotenspezifische Subjektmetadaten umfasst, wobei die Netzwerkkanten Konfigurationsmetadaten umfassen, wobei die Konfigurationsmetadaten Routingdaten umfassen, die zum Einrichten eines Kommunikationskanals zum Übermitteln von Daten zwischen zwei Netzwerkknoten des Netzwerkgraphen unter Verwendung einer Kommunikationsvorrichtung konfiguriert sind;
- Suchen (202) nach einem nächstgelegenen übereinstimmenden Netzwerkknoten (122), der aus den Netzwerkknoten ausgewählt wird, wobei der nächstgelegene übereinstimmende Netzwerkknoten durch Vergleichen der neuen Subjektmetadaten mit den knotenspezifischen Subjektmetadaten ausgewählt wird,
- Konstruieren (204) neuer Netzwerkkanten (126) für den neuen Knoten in dem Netzwerkgraphen, wobei die neuen Netzwerkkanten eine Replikation von mindestens einem Teil der Netzwerkkanten des nächstgelegenen übereinstimmenden Netzwerkknotens umfassen.

## Revendications

1. Système médical (100, 400) comprenant :
- une mémoire (110) stockant des instructions exécutables par machine (112) et un graphe de réseau (114, 114'), dans lequel le graphe de réseau comprend des nœuds de réseau (300) et des bords de réseau (302) qui relient les nœuds de réseau, dans lequel au moins une partie des nœuds de réseau comprend des métadonnées de sujet spécifiques à un nœud, dans lequel les bords de réseau comprennent des métadonnées de configuration, dans lequel les métadonnées de configuration comprennent des données de routage configurées pour établir un canal de communication pour communiquer des données entre deux nœuds de réseau du graphe de réseau à l'aide d'un dispositif de communication ;
- un système informatique (104), dans lequel une exécution des instructions exécutables par machine amène le système informatique à :
- créer (200) un nouveau nœud (116) comprenant de nouvelles métadonnées de sujet (118) dans le graphe de réseau ;
- rechercher (202) un nœud de réseau correspondant le plus proche (122) sélectionné parmi les nœuds de réseau, dans lequel le nœud de réseau correspondant le plus proche est sélectionné en comparant les nouvelles métadonnées de sujet aux métadonnées de sujet spécifiques à un nœud,
- construire (204) de nouveaux bords de réseau (126) pour le nouveau nœud dans le graphe de réseau, dans lequel les nouveaux bords de réseau comprennent une réplication d'au moins une partie des bords de réseau du nœud de réseau correspondant le plus proche.

2. Système médical selon la revendication 1, dans lequel le système médical comprend un système d'imagerie médicale (402) configuré pour acquérir des données d'imagerie médicale (450), dans lequel le système médical comprend le dispositif de communication, dans lequel les métadonnées spécifiques au sujet comprennent une classification d'image (438), dans lequel les métadonnées de configuration comprennent des données de configuration de dispositif adaptées pour commander le système d'imagerie médicale afin d'acquérir les données d'imagerie médicale, dans lequel une exécution des instructions exécutables par machine amène en outre le système informatique à :
- sélectionner (504) un bord de réseau choisi parmi l'un des nouveaux bords de réseau à l'aide de la classification d'image, dans lequel le nouveau nœud représente le dispositif de communication du système d'imagerie médicale, dans lequel le bord de réseau choisi est connecté à un bord de réseau supplémentaire représentant une entité distante (444) ;
- établir (506) le canal de communication entre le dispositif de communication et l'entité distante à l'aide des données de routage du nouveau bord de réseau, dans lequel le système médical est configuré de sorte que le canal de communication permette au moins une commande partielle du système d'imagerie médicale par l'entité distante ;
- commander (514) le système d'imagerie médicale afin d'acquérir les données d'imagerie médicale à l'aide des données de configuration de dispositif du bord de réseau choisi.

3. Système médical selon la revendication 2, dans lequel une exécution des instructions exécutables par machine amène en outre le système informatique à :
- envoyer (508) la classification d'image à l'entité distante via le canal de communication ;
- recevoir (510) des commandes de modification de configuration (448) de l'entité distante via le canal de communication ; et
- modifier (512) l'acquisition des données d'imagerie médicale à l'aide des commandes de modification de configuration.

4. Système médical selon la revendication 2 ou 3, dans lequel une exécution des instructions exécutables par machine amène en outre le système informatique à :
- commander (500) le système d'imagerie médicale afin d'acquérir des données d'imagerie médicale préliminaires (432) à l'aide de données de configuration préliminaires ;
- recevoir (502) la classification d'image en réponse à l'entrée des données d'imagerie médicale préliminaires dans un réseau neuronal de classification d'image (436).

5. Système médical selon l'une quelconque des revendications 2, 3 ou 4, dans lequel le système d'imagerie médicale est l'un quelconque parmi : un système d'imagerie par résonance magnétique (402), un système d'imagerie par ultrasons, un système à rayons X, un fluoroscope, un système de tomographie par émission de positrons, un système de tomographie par émission monophotonique et un système de tomodensitométrie.

6. Système médical selon l'une quelconque des revendications précédentes, dans lequel le système médical est un système de télémédecine.

7. Système médical selon l'une quelconque des revendications 1 à 5, dans lequel le système médical est un système d'information hospitalier ou une machine de diagnostic médical.

8. Système médical selon l'une quelconque des revendications précédentes, dans lequel les métadonnées de configuration comprennent l'un quelconque parmi : des processus de travail et des flux de travail.

9. Système médical selon l'une quelconque des revendications précédentes, dans lequel les métadonnées de configuration comprennent un indicateur indiquant si les métadonnées de configuration sont privées ou non privées, dans lequel une exécution des instructions exécutables par machine amène en outre le système informatique à empêcher la réplication des bords de réseau du nœud de réseau correspondant le plus proche s'ils présentent une indication privée.

10. Système médical selon l'une quelconque des revendications précédentes, dans lequel les métadonnées spécifiques au sujet comprennent un rôle professionnel et/ou des exigences fonctionnelles.

11. Système médical selon l'une quelconque des revendications précédentes, dans lequel une exécution des instructions exécutables par machine amène en outre le système informatique à :
- recevoir une sélection d'un nœud de réseau annulé, dans lequel le nœud de réseau annulé est l'un des nœuds de réseau ;
- envoyer un message préenregistré à des nœuds de réseau connectés au nœud de réseau annulé via le canal de communication pour des nœuds de réseau connectés au nœud de réseau annulé via des bords de réseau ; et
- supprimer le nœud de réseau annulé et les bords de réseau connectés au nœud de réseau annulé du graphe de réseau.

12. Système médical selon l'une quelconque des revendications précédentes, dans lequel les métadonnées de configuration comprennent des données de fréquence d'utilisation, dans lequel la réplication des bords de réseau du nœud de réseau correspondant avec des données de fréquence d'utilisation inférieures à un seuil d'utilisation prédéterminé est empêchée.

13. Système médical selon l'une quelconque des revendications précédentes, dans lequel une exécution des instructions exécutables par machine amène en outre le système informatique à :
- recevoir de multiples nœuds de réseau comprenant des métadonnées de sujet créées ;
- ajouter de manière itérative les multiples nœuds de réseau à un nouveau graphe de réseau, dans lequel l'ajout itératif des multiples nœuds de réseau comprend :
- l'ajout d'un des multiples nœuds de réseau au nouveau graphe de réseau,
- la copie des bords de réseau du graphe de réseau dans le nouveau graphe de réseau en faisant correspondre les métadonnées de sujet créées avec les métadonnées de sujet du graphe de réseau.

14. Procédé de fonctionnement d'un système médical (100, 400), dans lequel le système médical comprend une mémoire (110) stockant des instructions exécutables par machine (112) et un graphe de réseau (114, 114'), dans lequel le graphe de réseau comprend des nœuds de réseau (300) et des bords de réseau (302) qui relient les nœuds de réseau, dans lequel au moins une partie des nœuds de réseau comprend des métadonnées de sujet spécifiques au nœud, dans lequel les bords de réseau comprennent des métadonnées de configuration, dans lequel les métadonnées de configuration comprennent des données de routage configurées pour établir un canal de communication pour la communication de données entre deux nœuds de réseau du graphe de réseau à l'aide d'un dispositif de communication, dans lequel le procédé comprend :
- la création (200) d'un nouveau nœud (116) comprenant de nouvelles métadonnées de sujet (118) dans le graphe de réseau ;
- la recherche (202) d'un nœud de réseau correspondant le plus proche (122) sélectionné parmi les nœuds de réseau, dans lequel le nœud de réseau correspondant le plus proche est sélectionné en comparant les nouvelles métadonnées de sujet aux métadonnées de sujet spécifiques au nœud,
- la construction (204) de nouveaux bords de réseau (126) pour le nouveau nœud dans le graphe de réseau, dans lequel les nouveaux bords de réseau comprennent une réplication d'au moins une partie des bords de réseau du nœud de réseau correspondant le plus proche.

15. Produit programme informatique comprenant des instructions exécutables par machine (112) destinées à être exécutées par un système informatique (104), dans lequel lune exécution des instructions exécutables par machine amène le système informatique à :
- créer (200) un nouveau nœud (116) comprenant de nouvelles métadonnées de sujet (118) dans un graphe de réseau, dans lequel le graphe de réseau comprend des nœuds de réseau (300) et des bords de réseau (302) qui relient les nœuds de réseau, dans lequel au moins une partie des nœuds de réseau comprend des métadonnées de sujet spécifiques à un nœud, dans lequel les bords de réseau comprennent des métadonnées de configuration, dans lequel les métadonnées de configuration comprennent des données de routage configurées pour établir un canal de communication pour communiquer des données entre deux nœuds de réseau du graphe de réseau à l'aide d'un dispositif de communication ;
- rechercher (202) un nœud de réseau correspondant le plus proche (122) sélectionné parmi les nœuds de réseau, dans lequel le nœud de réseau correspondant le plus proche est sélectionné en comparant les nouvelles métadonnées de sujet aux métadonnées de sujet spécifiques au nœud,
- construire (204) de nouveaux bords de réseau (126) pour le nouveau nœud dans le graphe de réseau, dans lequel les nouveaux bords de réseau comprennent une réplication d'au moins une partie des bords de réseau du nœud de réseau correspondant le plus proche.
